(19) Europäisches Patentamt
European Patent Office
Office européen des brevets

(11) Veröffentlichungsnummer: **0 000 162**
**B1**

(12) **EUROPÄISCHE PATENTSCHRIFT**

(21) Anmeldenummer: **78100182.1**

(22) Anmeldetag: **19.06.78**

(51) Int. Cl.³: **C 07 C 43/20**, C 07 C 49/84, C 07 C 47/52, C 07 C 43/174, C 07 C 41/00, C 07 C 69/92, C 07 C 67/28

(54) **Verfahren zur Herstellung von Aralkyl- und Alkyl-phenol-äthern**

(30) Priorität: **28.06.77 DE 2729031**
**23.02.78 DE 2807762**

(43) Veröffentlichungstag der Anmeldung:
**10.01.79 Patentblatt 79/01**

(45) Bekanntmachung des Hinweises auf die Patenterteilung:
**20.08.80 Patentblatt 80/17**

(84) Benannte Vertragsstaaten:
**BE DE FR GB**

(56) Entgegenhaltungen:
**Chemical Abstracts, Vol. 77, 1972 T. Kato et al. "Aromatic ethers", Seite 465, Zusammenfassung Nummer 61549x**

(73) Patentinhaber: **BASF Aktiengesellschaft**
**Carl-Bosch-Strasse 38**
**D - 6700 Ludwigshafen (DE)**

(72) Erfinder: **Merger, Franz, Dr.**
**Max-Slevogt-Strasse 25**
**D - 6710 Frankenthal (DE)**
**Towae, Friedrich, Dr.**
**Parkstrasse 22**
**D - 6700 Ludwigshafen (DE)**
**Schroff, Ludwig, Dr.**
**verstorben, zuletzt wohnhaft Londoner Ring 72**
**D - 6700 Ludwigshafen (DE)**

**EP 0 000 162 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von Aralkyl- und Alkyl-phenol-äthern

Die Erfindung betrifft ein neues Verfahren zur Herstellung von Phenoläthern durch Umsetzung von Phenolen mit Dialkyl-carbonaten in Gegenwart tertiärer Amine und/oder Phosphine.

Es ist aus Houben-Weyl, Methoden der Organischen Chemie, Band 6/3, Seiten 54 bis 71, bekannt, daß Phenole mit Alkylestern anorganischer Säuren bzw. Chlorkohlensäureestern alkyliert werden können. Besondere Bedeutung haben dabei die Ester der Schwefelsäure und der Halogenwasserstoffe erlangt. Die Umsetzung wird bevorzugt in Genenwart von wäßriger oder alkoholischer Alkalilauge, Natriumcarbonat oder Kaliumcarbonat durchgeführt. Bei mehrwertigen Phenolen mit zwei metaständigen Hydroxygruppen, z.B Resorcin, muß in saurem Medium alkyliert werden, da sonst erhebliche Mengen an Kernalkylierungsprodukten entstehen (loc. cit., Seite 59 und 60). Allen diesen Verfahren haften jedoch gewisse Nachteile an. So wird bei der Alkylierung mit Estern starker anorganischer Säuren (z.B. Dimethylsulfat, Methyljodid) für jede eingeführte Alkylgruppe ein Äquivalent Säure freigesetzt, das durch Neutralisation wieder entfernt werden muß. Zudem sind viele dieser Alkylierungsmittel, z.B. Dimethylsulfat, außerordentlich toxisch. Die Verwendung von Alkalien ist besonders bei mehrwertigen Phenolen nachteilig, da deren Alkalienempfindlichkeit zu erheblichen Verlusten führt.

Auch die Umsetzung von Alkoholen selbst (loc. cit., Seiten 11 bis 18) ist technisch unbefriedigend, denn die Verwendung starker Säuren, wie Schwefelsäure, bringt Schwierigkeiten mit Bezug auf Korrosion der Anlageteile, schwierige Abtrennung des Katalysators und gegebenenfalls die Hydrolyseempfindlichkeit anderer funktioneller Gruppen, mit sich.

Es wurde nun gefunden, daß man Aralkyl- und Alkyl-phenol-äther der Formel

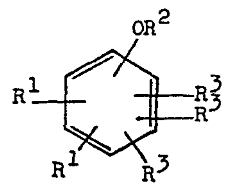 I,

worin die einzelnen Reste $R^1$ gleich oder verschieden sein können und jeweils ein Wasserstoffatom, eine Hydroxylgruppe oder den Rest —$OR^2$ bedeuten, oder beide Reste $R^1$ zusammen mit zwei einander benachbarten Kohlenstoffatomen des Benzolringes auch für einen aromatischen Rest stehen können, $R^2$ einen araliphatischen oder aliphatischen Rest bezeichnet, die einzelnen Reste $R^3$ gleich oder verschieden sein können und jeweils für einen aliphatischen, araliphatischen oder aromatischen Rest, ein Wasserstoffatom, ein Halogenatom, die Cyangruppe, die Nitrogruppe, den Rest

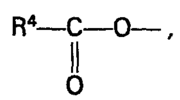

den Rest $R^4$—S—, den Rest $R^4$—O—, worin $R^4$ einen aliphatischen oder aromatischen Rest bedeutet, stehen, vorteilhaft erhält, wenn man Phenole der Formel

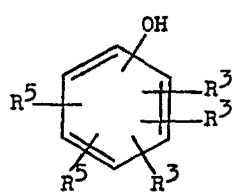 II,

worin $R^3$ die vorgenannte Bedeutung besitzt und $R^5$ die Bedeutung von $R^1$ besitzt oder, wenn $R^1$ den Rest —$OR^2$ bedeutet, auch jeweils eine Hydroxylgruppe bezeichnen kann, mit Diaralkylcarbonaten oder Dialkylcarbonaten der Formel

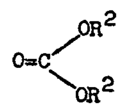 III,

worin die einzelnen Reste $R^2$ gleich oder verschieden sein können und die vorgenannte Bedeutung besitzen, in Gegenwart eines tertiären Amins und/oder Phosphins bei einer Temperatur oberhalb 100°C umsetzt.

Die Umsetzung kann für den Fall der Verwendung von Phenol und Dimethylcarbonat durch die folgenden Formeln wiedergegeben werden:

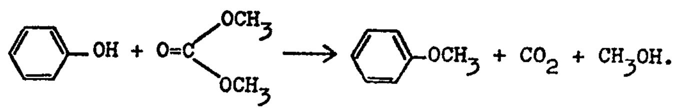

Im Vergleich zu den bekannten Verfahren liefert das Verfahren nach der Erfindung auf einfacherem und wirtschaftlicherem Wege eine große Zahl von Phenoläthern in guter Ausbeute und Reinheit. Umständliche Abtrennungs- und Neutralisations-operationen sowie Korrosionsprobleme werden vermieden.

Außer dem Endstoff werden lediglich ein Mol Alkohol sowie das nicht toxische Kohlendioxid erhalten; das erfindungsgemäße Verfahren ist somit umweltfreundlicher als die bekannten Verfahren. Der freiwerdende Alkohol kann in bekannter Weise ohne Phosgen wieder mit CO und Sauerstoff zu Dialkylcarbonat bzw. Diaralkylcarbonat

$$2\ R^2OH + CO + \frac{1}{2}\ O_2 \longrightarrow O{=}C(OR^2)_2 + H_2O$$

verarbeitet werden (deutsche Offenlegungsschrift 2 334 736). Alle diese vorteilhaften Eigenschaften des erfindungsgemäßen Verfahrens sind im Hinblick auf den Stand der Technik überraschend. Auch hätte man wirksame Katalysatoren für die Verätherung gerade nicht in tertiären Aminen und insbesondere Pyridinen, die üblicherweise als Acylierungskatalysatoren verwendet werden, erwartet.

Die Dialkylcarbonate können in bekannter Weise, z.B. nach den in der deutschen Offenlegungsschrift 2 160 111 angegebenen Verfahren oder zweckmäßiger, insbesondere aus Gründen des Umweltschutzes, durch Umsetzung eines Alkohols mit Kohlenmonoxid und Sauerstoff in Gegenwart von Kupferkatalysatoren nach dem in der deutschen Offenlegungsschrift 2 334 736 beschriebenen Verfahren, hergestellt werden. Die Ausgangsphenole können 3 oder 2 Hydroxylgruppen und bevorzugt eine umsetzbare Hydroxylgruppe tragen. Bevorzugte Ausgangsstoffe II und III und dementsprechend bevorzugte Endstoffe I sind solche, in deren Formeln die einzelnen Reste $R^1$ gleich oder verschieden sein können und jeweils ein Wasserstoffatom, eine Hydroxylgruppe oder den Rest $—OR^2$ bedeuten, oder beide Reste $R^1$ zusammen mit zwei einander benachbarten Kohlenstoffatomen des Benzolringes auch für einen anellierten Phenylenrest stehen können, die einzelnen Reste $R^2$ gleich oder verschieden sein können und jeweils einen Aralkylrest mit 7 bis 12 Kohlenstoffatomen oder insbesondere einen Alkylrest mit 1 bis 7, zweckmäßig 1 bis 4 Kohlenstoff atomen bezeichnet, die einzelnen Reste $R^3$ gleich oder verschieden sein können und jeweils für einen Phenylrest, einen Aralkylrest mit 7 bis 12 Kohlenstoffatomen oder insbesondere einen Alkylrest mit 1 bis 12, zweckmäßig 1 bis 6 Kohlenstoffatomen, einen Alkenylrest mit 2 bis 12, zweckmäßig mit 2 bis 6 Kohlenstoffatomen und bevorzugt einer Doppelbindung, einen durch 2 Oxogruppen substituierten Alkylrest mit 1 bis 12 Kohlenstoffatomen, zweckmäßig 1 bis 6 Kohlenstoffatomen, ein Wasserstoffatom, ein Bromatom, ein Chloratom, die Cyangruppe, die Nitrogruppe, den Rest

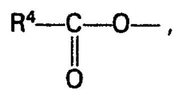

den Rest $R^4—S—$, den Rest

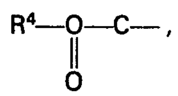

den Rest $R^4—O—$, worin $R^4$ einen Alkylrest mit 1 bis 12, zweckmäßig 1 bis 6 Kohlenstoffatomen oder einen Phenylrest bedeutet, stehen, $R^5$ die Bedeutung von $R^1$ besitzt oder, wenn $R^1$ de Rest $—OR^2$ bedeutet, auch jeweils eine Hydroxylgruppe bezeichnen kann. Die vorgenannten Reste können noch durch unter den Reaktionsbedingungen inerte Gruppen, z.B. Alkylgruppen oder Alkoxygruppen mit jeweils 1 bis 4 Kohlenstoffatomen, substituiert sein. Als aliphatische Reste werden auch solche bezeichnet, deren Alkylrest durch Oxogruppen substituiert sind, z.B. Acyl-, Aldehydo- und Carbalkoxygruppen.

Die Ausgangsstoffe II werden mit den Ausgangsstoffen III in stöchiometrischer Menge oder im Uberschuß oder Unterschuß umgesetzt, vorteilhaft im Falle der Umsetzung von Monohydroxyverbindungen II von 1 bis 10 Mol, insbesondere von 1,2 bis 5 Mol Ausgangsstoff III je Mol Ausgangsstoff II oder im Falle der Umsetzung von Dihydroxyverbindungen II zu Monohydroxymonoätherverbindungen I von 0,1 bis 2, insbesondere von 0,2 bis 1 Mol Ausgangsstoff III je Mol Ausgangsstoff II oder im Falle der Umsetzung von Dihydroxyverbindungen II zu Diäther I von 2 bis 10, insbesondere von 2 bis 5 Mol Ausgangsstoff III je Mol Ausgangsstoff II oder im Falle der Umsetzung von Trihydroxyverbindungen II zu Dihydroxymonoätherverbindungen I von 0,1 bis 2, insbesondere von 0,2 bis 1 Mol Ausgangsstoff III je Mol Ausgangsstoff II oder im Falle der Umsetzung von Trihydroxyverbindungen II zu Monohydroxydiätherverbindungen I von 2,01 bis 4, insbesondere von 2,1 bis 3 Mol Ausgangsstoff III je Mol Ausgangsstoff II oder im Falle der Umsetzung von Trihydroxyverbindungen II zu Triäther I von 4,01 bis 15, insbesondere von 4,1 bis 10 Mol Ausgangsstoff III je Mol Ausgangsstoff II.

Es kommen als Ausgangsstoffe II beispielsweise in Frage: Phenol; in 2-Stellung, 3-Stellung, 4-Stellung, 5-Stellung und/oder 6-Stellung einfach, zweifach, dreifach oder vierfach mit gleichen oder verschiedenen Substituenten durch die Methyl-, Athyl-, n-Propyl-, Isopropyl-, n-Butyl-, Isobutyl-, sek.-Butyl-, tert.-Butyl-, Benzyl-, Phenyl-, Cyan-, Nitro-, Formyl-, Acetyl-, Propionyl-, Butyryl-gruppe, durch Brom, Chlor, durch die $\beta$-Formyläthyl-, $\gamma$-Formylpropyl-, $\delta$-Formylbutyl, $\beta$-Acetyläthyl-, $\gamma$-Acetylpropyl-, $\delta$-Acetylbutyl-, $\beta$-Propionyläthyl-, $\gamma$Propionylpropyl-, $\delta$-Propionylbutyl-, Methylcarbonyloxy-, Äthylcarbonyloxy-, Propylcarbonyloxy-, Isopropylcarbonyloxy-, Butylcarbonyloxy-, Isobutylcarbonyloxy-, sek.-Butylcarbonyloxy-, tert.-Butylcarbonyloxy-, Phenylcarbonyloxy-, Carbmethoxy-, Carbäthoxy-, Carbpropoxy-, Carbisopropoxy-, Carbbutoxy-, Carbisobutoxy-, Carb-sek.-butoxy-, Carb-tert.-butoxy-, Carbphenoxy-, Methylthio-, Äthylthio-, Propylthio-, Isopropylthio-, Butylthio-, Isobutylthio-, sek.-Butylthio-, tert.-Butylthio-, Phenylthio-, Methoxy-, Isopropoxy-, Butoxy-, Isobutoxy-, sek.-Butoxy-, tert.-Butoxy-, tert.-Butoxy-gruppe substituiertes Phenol; Brenzcatechin, Hydrochinon, Resorcin und entsprechende, in den übrigen Stellungen durch vorgenannte Gruppen einfach, zweifach, dreifach oder vierfach substituierte Brenzcatechine, Hydrochinone und Resorcine; $\alpha$- und $\beta$-Naphthol und analog mit vorgenannten Substituenten substituierte Naphthole; Pyrogallol, Phloroglucin, 1,3,4-Trihydroxybenzol und analog substituierte Trihydroxybenzole.

Bevorzugte Ausgangsstoffe ii sind: Phenol, o-, m-, p-Kresol, o-, m-, p-Äthylphenol, 2,3-, 2,4-, 2,5-, 2,6-, 3,5-, 3,4-Xylenol, o-, m-, p-Isopropylphenol, 2,4-, 2,6-Dimethylphenol, 2,4,6-Trimethylphenol, 2-Methyl-5-isopropylphenol, 3-Methyl-6-isopropylphenol, o-, m-, p-tert.-Butylphenol, p-tert.-Butylphenol, Octylphenol, Nonylphenol, Dodecylphenol, 2,6-, 2,4-Di-tert.-butylphenol, 3-methyl-4,6-di-tert.-butylphenol, Salicylalkohol, Salicylaldehyd, Salicylsäuremethylester, Vanillin, Gallussäuremethylester, Eugenol, Isoeugenol, Chavibetol, $\beta$-(4-Hydroxyphenyl)-äthylmethylketon, $\alpha$-Naphthol, $\beta$-Naphthol, 2-, 3-, 4-Bromphenol, 2-, 4-Nitrophenol, 3-Brom-2,4-dinitrophenol, 4-Brom-2,6-dinitrophenol, 4-Acetylphenol, 2-, 4-Methylmercaptophenol, Brenzkatachin, 4-tert.-Butylbrenzkatechin, Resorcin, 5-Methylresorcin, 4,6-Dimethylresorcin, Hydrochinon, tert.-Butylhydrochinon, 2-, 3-, 4-Methoxyphenol, 2,4-Di-tert.-butyl-6-methylphenol, 2,6-Di-tert.-butyl-4-methylphenol, 2,5-, 2,6-Di-tert.-butyl-hydrochinon.

Als Ausgangsstoffe III kommen in Betracht: Dimethylcarbonat, Diäthylcarbonat, Di-n-propylcarbonat, Di-n-butylcarbonat, Diisobutylcarbonat, Dibenzylcarbonat, Methyläthylcarbonat, Methylpropylcarbonat, Äthylpropylcarbonat; bevorzugt sind Dimethylcarbonat, Diäthylcarbonat, Di-n-propylcarbonat, Methyläthylcarbonat, Dibenzylcarbonat.

Die Umsetzung wird bei einer Temperatur oberhalb 100°C, im allgemeinen zwischen 100°C und 350°C, vorzugsweise von 120 bis 300°C, im Falle von Monohydroxyverbindungen II vorteilhaft von 120 bis 200, insbesondere von 130 bis 180°C; im Falle der Herstellung von Monoätherverbindungen I, ausgehend von Dihydroxyverbindungen II oder Trihydroxyverbindungen II, vorteilhaft von 100 bis 170, insbesondere von 110 bis 150°C; im Falle der Herstellung von Diäther I und Triäther I, ausgehend von Dihydroxyverbindungen II oder Trihydroxyverbindungen II, vorteilhaft von 120 bis 200, insbesondere von 140 bis 180°C, drucklos oder unter Druck, vorzugsweise unter dem im Autoklaven bei vorgenannten Temperaturen sich einstellenden Dampfdruck des Reaktionsgemisches, zweckmäßig von 1 bis 200 bar, kontinuierlich oder diskontinuierlich durchgeführt. Zweckmäßig dient das Reaktionsgemisch gleichzeitig als Lösungsmedium bzw. Suspensionsmedium. In solchen Fällen istes bisweilen vorteilhaft, einen Überschuß an Ausgangsstoff III und/oder schon am Anfang eine Zusatzmenge von dem bei der Reaktion sich bildenden Alkohol zuzugeben. Gegebenenfalls können, insbesondere bei Herstellung der Monohydroxyverbindungen aus den Dihydroxyverbindungen, unter den Reaktionsbedingungen inerte Lösungsmittel verwendet werden. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin, Chlorbenzol, o- und m-Dichlorbenzol, o-, m-, p-Chlortoluol, 1,2,4-Trichlorbenzol; Äther, z.B. Äthylpropyläther, Methyl-tert.-butyläther, n-Butyläthyläther, Di-n-butyläther, Diisobutyläther, Diisoamyläther, Diisopropyläther, Anisol, Phenetol, Cyclohexylmethyläther, Diäthyläther, Äthylenglykoldimethyläther, Tetrahydrofuran, Dioxan, $\beta$,$\beta$-Dichlordiäthyläther; aliphatische oder cycloaliphatische Kohlenwasserstoffe, z.B. Heptan, Nonan, Benzinfraktionen innerhalb eines Siedepunktintervalls von 70 bis 190°C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan; und entsprechende Gemische. Zweckmäßig verwendet man das Lösungsmittel in einer Menge von 100 bis 2 000 Gewichtsprozent, bezogen auf Ausgangsstoff III.

Man verwendet als Katalysator ein tertiäres Amin, vorteilhaft in einer Menge von 0,1 bis 10, insbesondere von 1 bis 5 Molprozent, bezogen auf Ausgangsstoff II. Auch Gemische der genannten Katalysatoren kommen für die Reaktion in Betracht. Das Amin kann auch in Gestalt von Diaminen, entsprechenden Salzen oder einem quaternären Salz verwendet werden. Geeignete Katalysatoren sind Trimethylamin, Dimethylamino-neopentanol, N,N'-Tetramethyldiamino-neopentan, Äthyldimethylamin, Lauryldimethylamin, Stearyldimethylamin, Pyridin, $\alpha$-, $\beta$-, und $\gamma$-Picolin, Chinolin, Isochinolin, Chinazolin, Chinoxalin, Amyldimethylamin, Propyldimethylamin, Butyldimethylamin, N-Methylimidazol, N-Methylpyrrol, 2,6-, und 2,4-Lutidin, Triäthylendiamin, p-Dimethylaminopyridin, N,N-Dimethylcyclohexylamin, Pyrimidin, Acridin; Di-(methyl)-, Di(äthyl)-, Di-(n-propyl)-, Di-(n-butyl)-, Di-(pentyl)-, Di-(n-hexyl)-, Di-(n-heptyl)-, Di-(n-octyl)-, Di-(n-nonyl)-, Di-(n-decyl)-anilin und mit vorgenannten Resten am Stickstoffatom einfach substituiertes Pyrazolidin, Imidazolidin, Morpholin, Piperidin, Pyrrolidin; entsprechende Katalysatoren mit 2 oder 3 vorgenannten, aber unterschiedlichen Resten, z.B. Dimethyläthylamin. Ebenfalls kommen auch tertiäre Aminogruppen enthaltende Polymere, z.B. 4-Polyvinylpyridin und Polyvinylimidazol-(N), in Frage.

Besonders vorteilhaft sind Trimethylamin, Pyridin, $\gamma$-Picolin, 4-Pyrrolidino-(1')-pyridin und andere Pyridinderivate wie o-Methyl-, m-Methyl-, o-Äthyl-, m-Äthyl-, p-Äthyl-, o-Propyl-, m-Propyl-, p-Propylpyridin, Dimethylamino-neopentanol, N,N'-Tetramethyldiamino-neopentan, p-Methoxy-, p-Äthoxy-, p-Propoxy-pyridin, p-Dimethylaminopyridin, p-Diäthyl aminopyridin, p-Dipropylaminopyridin, 4-Polyvinylpyridin.

Man verwendet ebenfalls als Katalysator ein tertiäres Phosphin, vorteilhaft in einer Menge von 0,1 bis 10, insbesondere von 1 bis 5 Molprozent tertiärem Phosphin, bezogen auf Ausgangsstoff II. Auch Gemische der genannten Katalysatoren kommen für die Reaktion in Betracht. Das Phosphin kann auch in Gestalt von Diphosphin verwendet werden. Geeignete Katalysatoren sind solche der Formel

$$\overset{R^6}{\underset{R^6}{P}}-R^6 \quad \text{IV} \qquad \text{oder } R^6-\overset{R^6}{P}-R^7-\overset{R^6}{P}-R^6 \quad \text{V},$$

worin die einzelnen Reste $R^6$ gleich oder verschieden sein können und jeweils einen aliphatischen oder aromatischen Rest, vorzugsweise einen Alkylrest mit 1 bis 6 Kohlenstoffatomen, einen durch mehrere Cyangruppen oder bevorzugt eine Cyangruppe substituierten Alkylrest mit 2 bis 9 Kohlenstoffatomen, einen Phenylrest oder einen durch eine oder 2 Alkylgruppen mit jeweils 1 bis 4 Kohlenstoffatomen und/oder eine oder 2 Alkoxygruppen mit jeweils 1 bis 4 Kohlenstoffatomen substituierten Phenylrest bedeuten, $R^7$ einen aliphatischen Rest vorzugsweise einen Alkylenrest mit 1 bis 6 Kohlenstoffatomen bezeichnet. Die vorgenannten Reste können noch durch unter den Reaktionsbedingungen inerte Gruppen, z.B. durch Alkylgruppen oder Alkoxygruppen mit jeweils 1 bis 4 Kohlenstoffatomen, Carboxygruppen mit 2 bis 4 Kohlenstoffatomen, substituiert sein. Beispielsweise kommen folgende Phosphine IV und V als Katalysatoren in Frage: Trimethylphosphin, Triäthylphosphin, Tripropylphosphin, Triisopropylphosphin, Tributylphosphin, Triisobutylphosphin, Tri-sek.-butylphosphin, Triphenylphosphin; P,P-Dimethyl-P-neopentylphosphin, P-Äthyl-P,P-dimethylphosphin, P-Lauryl-P,P-dimethylphosphin, P-Stearyl-P,P-dimethylphosphin, P-Amyl-P,P-dimethylphosphin, P-Propyl-P,P-dimethylphosphin, P,P-Butyl-P-dimethylphosphin, P,P-Dimethyl-P-phenylphosphin, Tri-(pentyl)-phosphin, Tri-(n-hexyl)-phosphin, Tri-(n-heptyl)-phosphin, Tri-(n-octyl)-phosphin, Tri-(n-nonyl)-phosphin, Tri-(n-decyl)-phosphin, Tri-(2-Methoxy)-phenylphosphin, Tri-o-tolylphosphin, Tri-m-tolyphosphin, Tri-p-tolylphosphin; Tri-o-xylylphosphine, Tri-m-xylylphosphine, Tri-p-xylylphosphine, wobei Phosphor vorteilhaft in m-Stellung zu einer der beiden Methylgruppen steht; P,P-Bis-(2-cyanoäthyl)-P-phenylphosphin, Tri-(2-cyanoäthyl)-phosphin, Tri-(carbäthoxymethyl)-phosphin, P-Carbäthoxymethyl-P,P-diäthyl-phosphin, P,P-Diäthyl-P-($\beta$-carbäthoxyäthyl)-phosphin P-Carbäthoxymethyl-P,P-diphenyl-phosphin; Bis-(diphenylphosphino)-alkane mit einer Aklylengruppe mit 1 bis 6 Kohlenstoffatomen, z.B. 1,2-Bis-(diphenylphosphino)-äthan; Bis-(äthylphenylphosphino)-alkane mit einer Alkylengruppe mit 1 bis 6 Kohlenstoffatomen, z.B. 1,2-Bis-(äthyl-phenyl-phosphino)-äthan, 1,4-Bis-(äthyl-phenyl-phosphino)-butan; Bis-(dialkylphosphino)-alkane mit einer Alkylengruppe mit 1 bis 6 Kohlenstoffatomen und Alkylgruppen mit 1 bis 4 Kohlenstoffatomen, z.B. 1,5-Bis-(diäthylphosphino)-pentan; P-Propyl-P-hexyl-P-nonyl-phosphin, P-Äthyl-P-(2-äthoxyäthyl)-P-phenylphosphin, P-Isopropyl-P,P-diphenyl-phosphin, P-Butyl-P,P-diphenylphosphin.

Die Umsetzung kann wie folgt durchgeführt werden: Ein Gemisch der Ausgangsstoffe II, III, des Katalysators und gegebenenfalls des Lösungsmittels wird während 1 bis 20 Stunden bei der Reaktionstemperatur gehalten. Aus dem Gemisch wird dann der Endstoff in üblicher Weise, z.B. durch fraktionierte Destillation, abgetrennt.

Die nach dem Verfahren der Erfindung herstellbaren Aralkylaryl- und Alkylaryläther sind wertvolle Ausgangsstoffe für die Herstellung von Farbstoffen, Pflanzenschutzmitteln und Riechstoffen. Bezüglich der Verwendung wird auf vorgenannte Veröffentlichungen und Ullmanns Encyklopädie der technischen Chemie, Band 13, Seiten 450 bis 453, und Band 14, Seiten 760 bis 763, verweisen.

Die in den folgenden Beispielen aufgeführten Teile bedeuten Gewichtsteile.

## Beispiel 1

19 Teile Phenol werden mit 54 Teilen Dimethylcarbonat und 1 Teil p-Dimethylaminopyridin 10 Stunden bei 180°C gehalten. Anschließend wird unumgesetztes Dimethylcarbonat abdestilliert und das Anisol vom Rückstand durch Fraktionieren abgetrennt. Man erhält 21,4 Teile (98% der Theorie, bezogen auf umgesetzten Ausgangsstoff II) Anisol vom Kp 154°C. Der Umsatz ist praktisch quantitativ.

## Beispiel 2

22 Teile Hydrochinon werden mit 18 Teilen Dimethylcarbonat und 1 Teil Pyrrolidinopyridin in 54 Teilen Dioxan 15 Stunden bei 150°C gehalten. Die Aufarbeitung erfolgt analog Beispiel 1. Man erhält 8,0 Teile (98% der Theorie, bezogen auf umgesetzten Ausgangsstoff II) Hydrochinon-monomethyläther vom Fp 57°C. Der Umsatz beträgt 33 Prozent.

Beispiel 3

44,4 Teile 2,5-Di-tert.-butylhydrochinon werden mit 18 Teilen Dimethylcarbonat und 1 Teil p-Dimethylaminopyridin 20 Stunden bei 200°C gehalten. Die Aufarbeitung erfolgt analog Beispiel 1. Man erhält 9,5 Teile (26% der Theorie, bezogen auf umgesetzten Ausgangsstoff II) 2,5-Di-tert.-butylhydrochinon-dimethyläther vom Kp 71 bis 74°C/0,5 mbar und 22,4 Teile (65% der Theorie) 2,5-Di-tert.-butylhydrochinon-monomethyläther vom Kp 77 bis 79°C/0,5 mbar. Der Umsatz beträgt 73 Prozent.

Beispiel 4

22 Teile Resorcin werden mit 18 Teilen Dimethylcarbonat und 0,5 Teilen p-Dimethylaminopyridin 10 Stunden bei 180°C gehalten. Die Aufarbeitung erfolgt analog Beispiel 1. Man erhält 2,3 Teile (20% der Theorie, bezogen auf umgesetzten Ausgangsstoff II) Resorcin-dimethyläther vom Kp 214°C und 9,1 Teile (80% der Theorie) Monomethyläther vom Kp 245°C. Der Umsatz beträgt 46 Prozent.

Beispiel 5

33 Teile Eugenol werden mit 50 Teilen Dimethylcarbonat und 0,5 Teilen p-Dimethylaminopyridin 20 Stunden bei 200°C gehalten. Die Aufarbeitung erfolgt analog Beispiel 1. Man erhält 28 Teile (99% der Theorie, bezogen auf umgesetzten Ausgangsstoff II) Eugenolmonomethyläther vom Kp 69 bis 70°C (0,13 mbar). Der Umsatz beträgt 80 Prozent.

Beispiel 6

36 Teile 4-tert.-Butylbrenzkatechin werden mit 40 Teilen Dimethylcarbonat und 1 Teil p-Dimethylaminopyridin 10 Stunden bei 180°C gehalten. Die Aufarbeitung erfolgt analog Beispiel 1. Man erhält 18,2 Teile (49% der Theorie, bezogen auf umgesetzten Ausgangsstoff II) 4-tert.-Butyl-2-hydroxyanisol und 17 Teile (46% der Theorie) 5-tert.-Butyl-2-hydroxyanisol vom Kp 68 bis 70°C (0,13 mbar). Der Umsatz beträgt 95 Prozent.

Beispiel 7

33 Teile β-(4-Hydroxyphenyl)-äthylmethylketon werden mit 50 Teilen Dimethylcarbonat und 0,5 Teilen 4-Polyvinylpyridin 10 Stunden bei 170°C gehalten. Die Aufarbeitung erfolgt analog Beispiel 1. Man erhält 34,9 Teile (98% der Theorie, bezogen auf umgesetzten Ausgangsstoff II) β-(4-Methoxyphenyl)-äthylmethylketon vom Kp 94 bis 95°C (0,26 mbar). Der Umsatz ist praktisch quantitativ.

Beispiele 8 bis 19

Sie werden analog Beispiel 1 durchgeführt (Tabelle).

TABELLE

| Beispiel Nr. | Teile | Ausgangsstoff | Teile | Endprodukt | Kp in °C | Ausbeute in % der Theorie | Umsatz in % |
|---|---|---|---|---|---|---|---|
| 8 | 23,6 | 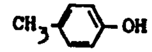 | 17,4 | 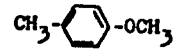 | 177 | 96 | 68 |
| 9 | 24,5 | 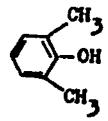 | 10,9 | 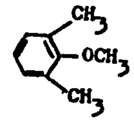 | 182 | 93 | 43 |
| 10 | 24,5 | 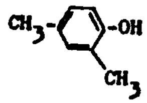 | 9,1 | 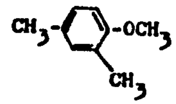 | 192 | 96 | 35 |
| 11 | 27,2 | 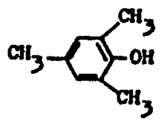 | 8,7 | 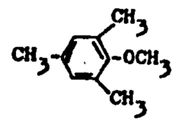 | 202 | 100 | 29 |
| 12 | 34,6 | 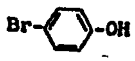 | 12,3 | 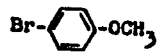 | 223 | 100 | 33 |
| 13 | 27,8 | 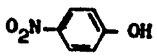 | 10,5 | 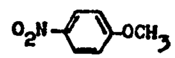 | 274 | 98 | 35 |
| 14 | 44 | 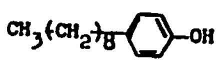 | 22,7 | 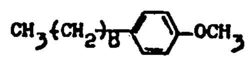 | 176–180/24 mbar | 78 | 66 |
| 15 | 27,3 | 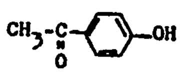 | 18,3 | 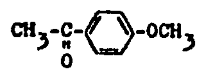 | 256 | 95 | 64 |
| 16 | 22 | 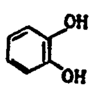 | 6,3 | 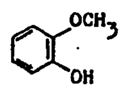 | 205 | 88 | 29 |
| 17 | 30,5 | 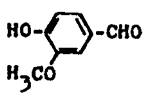 | 2,3 | 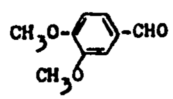 | 154–155/13 mbar | 12 | 58 |
| 18 | 28,8 | 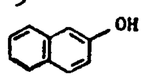 | 16,5 | 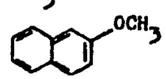 | 274 | 93 | 56 |
| 19 | 28,8 | 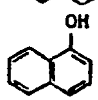 | 17,1 | 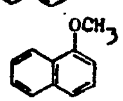 | 269 | 89 | 61 |

Beispiel 20

Die Umsetzung wird analog Beispiel 1 mit 18,4 Teilen Gallussäuremethylester und 80 Teilen Dimethylcarbonat durchgeführt. Man erhält 16,5 Teile (76% der Theorie) 3,4,5-Trimethoxybenzoesäuremethylester vom Kp 60 bis 63°C/0,07 mbar. Der Umsatz beträgt 96 Prozent.

Beispiel 21

Die Umsetzung wird analog Beispiel 1 mit 25,2 Teilen Pyrogallol und 70 Teilen Dimethylcarbonat durchgeführt. Man erhält 27,9 Teile (83% der Theorie) 1,2,3-Trimethoxybenzol vom Kp 70 bis 72°C/0,6 mbar. Der Umsatz ist praktisch quantitativ.

Beispiel 22

Die Umsetzung wird analog Beispiel 1 mit 22,8 Teilen 5-Methylresorcin und 9 Teilen Dimethylcarbonat durchgeführt. Man erhält 9 Teile (98%) der Theorie) 5-methylresorcinmonomethyläther vom Kp 63 bis 65°C/0,4 mbar. Der Umsatz beträgt 36 Prozent.

Beispiel 23

19 Teile Phenol werden mit 18 Teilen Dimethylcarbonat und einem Teil Tri-n-butylphosphin in 40 Teilen Methanol 10 Stunden bei 180°C gehalten Anschließend werden unumgesetztes Dimethylcarbonat und Lösungsmittel abdestilliert und das Anisol vom Rückstand durch Fraktionieren abgetrennt. Man erhält 14,2 Teile (99% der Theorie, bezogen aur umgesetztes Phenol) Anisol vom Kp 154°C. Der Umsatz beträgt 65 Prozent.

Beispiel 24

33 Teile $\beta$-(4-Hydroxyphenyl)-äthylmethylketon werden mit 54 Teilen Dimethylcarbonat und einem Teil Triphenylphosphin 10 Stunden bei 170°C gehalten. Man erhält 23,9 Teile (96% der Theorie, bezogen auf umgesetzten Ausgangsstoff II) $\beta$-(4-Methoxyphenyl)-äthylmethylketon vom Kp 94 bis 95°C (0,26 mbar). Der Umsatz beträgt 69,5 Prozent.

Beispiel 25

33 Teile Eugenol werden mit 50 Teilen Dimethylcarbonat und 2 Teilen Tri-n-butylphosphin 15 Stunden bei 190°C gehalten. Die Aufarbeitung erfolgt analog Beispiel 24. Man erhält 31 Teile (88% der Theorie, bezogen auf umgesetztes Eugenol) Eugenolmethyläther vom Kp 69 bis 70°C (0,13 mbar). Der Umsatz ist praktisch quantitativ.

Beispiel 26

22 Teile Hydrochinon werden mit 18 Teilen Dimethylcarbonat und einem Teil Triphenylphosphin 15 Stunden bei 190°C gehalten. Die Aufarbeitung erfolgt analog Beispiel 23. Man erhält 3 Teile (97% der Theorie, bezogen auf umgesetztes Hydrochinon) Hydrochinonmonomethyläther vom Fp 57°C. Der Umsatz beträgt 12 Prozent.

**Patentanspruch**

Verfahren zur Herstellung von Aralkyl- und Alkyl-phenyl-äthern der Formel

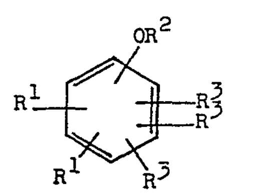 I,

worin die einzelnen Reste $R^1$ gleich oder verschieden sein können und jeweils ein Wasserstoffatom, eine Hydroxylgruppen oder den Rest —$OR^2$ bedeuten, oder beide Reste $R^1$ zusammen mit zwei einander benachbarten Kohlenstoffatomen des Benzolringes auch für einen aromatischen Rest stehen können, $R^2$ einen araliphatischen oder aliphatischen Rest bezeichnet, die einzelnen Reste $R^3$ gleich oder verschieden sein können und jeweils für einen aliphatischen, araliphatischen, oder aromatischen Rest, ein Wasserstoffatom, ein Halogenatom, die Cyangruppe, die Nitrogruppe, den Rest

$$R^4—\underset{\underset{O}{\|}}{C}—O—,$$

den Rest $R^4$—S—, den Rest $R^4$—O—, worin $R^4$ einen aliphatischen oder aromatischen Rest bedeutet, stehen, *dadurch gekennzeichnet,* daß man Phenole der Formel

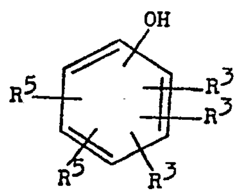 II,

worin $R^3$ die vorgenannte Bedeutung besitzt und $R^5$ die Bedeutung von $R^1$ besitzt oder, wenn $R^1$ den Rest $—OR^2$ bedeutet, auch jeweils eine Hydroxylgruppe bezeichnen kann, mit Diaralkylcarbonaten oder Dialkylcarbonaten der Formel

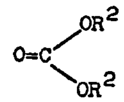 III,

worin die einzelnen Reste $R^2$ gleich oder verschieden sein können und die vorgenannte Bedeutung besitzen, in Gegenwart eines tertiären Amins und/oder tertiären Phosphins, wobei das Phosphin auch in Gestalt von Diphosphin verwendet werden kann, bei einer Temperatur oberhalb 100°C umsetzt.

**Revendication**

Procédé de préparation d'éthers aralkyliques et alkylphényliques répondant à la formule

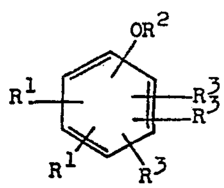 I,

dans laquelle les divers symboles $R^1$, ayant des significations identiques ou difféventes, représentent chacun un atome d'hydrogène, un groupe hydroxy ou le groupe $—OR^3$, ou bien les deux symboles $R^1$ forment ensemble et avec deux atomes de carbone voisins du cycle benzénique, un reste aromatique, $R^2$ représente un reste araliphatique, les divers symboles $R^3$, ayant des significations identiques ou différentes, représentent chacun un reste aliphatique, araliphatique ou aromatique, un atome d'hydrogène, un atome d'halogène, le groupe cyano, le groupe nitro, le groupe

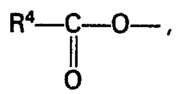

le groupe $R^4—S—$, le groupe $R^4—O—$, $R^4$ représentant un reste aliphatique ou aromatique, ce procédé se caractérisant en ce que l'on fait réagir des phénols répondant à la formule

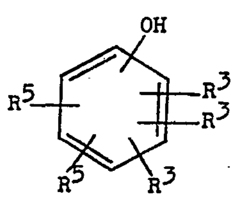 II,

dans laquelle les symboles $R^3$ ont les significations indiquées ci-dessus et les symboles $R^5$ ont les mêmes significations que $R^1$ ou bien, lorsque $R^1$ représente le groupe $—OR^2$, peuvent également représenter chacun un groupe hydroxy, avec des carbonates de diaralkyle ou des carbonates de dialkyle répondant à la formule

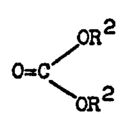 III,

dans laquelle les divers symboles $R^2$, ayant des significations identiques ou différentes, ont les significations indiquees ci-dessus, en présence d'une amine tertiaire et/ou d'une phosphine tertiaire, la phosphine pouvant également être utilisée à l'état de diphosphine, à une température supérieure à 100°C.

**Claim**

A process for the manufacture of an aralkyl phenyl ether or alkyl phenyl ether of the formula

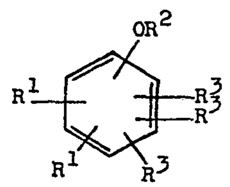

I,

where the radicals $R^1$ are identical or different and each is hydrogen, hydroxyl or $—OR^2$, or the two radicals $R^1$ together with two mutually adjoining carbon atoms of the benzene ring are an aromatic radical, $R^2$ is an araliphatic or aliphatic radical and the radicals $R^3$ are identical or different and each is an aliphatic, araliphatic or aromatic radical, hydrogen halogen, cyano, nitro,

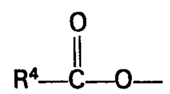

,

$R^4—S—$ or $R^4—O—$, where $R^4$ is an aliphatic or aromatic radical, *characterized in that* a phenol of the formula

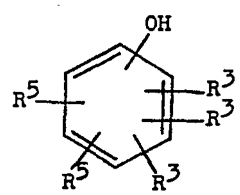

II,

where $R^3$ has the above meanings and $R^5$ has the meanings of $R^1$ or, if $R^1$ is $—OR^2$, may also be hydroxyl, is reacted with a diaralkyl carbonate or a dialkyl carbonate of the formula

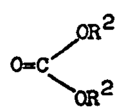

III,

where the radicals $R^2$, are identical or different and have the above meanings, in the presence of a tertiary amine and/or of a tertiary phosphine, the phosphine also being suitable in the form of diphosphine, at above 100°C.